(19) Europäisches Patentamt
European Patent Office
Office européen des brevets

(11) **EP 4 588 435 A1**

(12) **EUROPEAN PATENT APPLICATION**

(43) Date of publication:
**23.07.2025 Bulletin 2025/30**

(21) Application number: **24152828.0**

(22) Date of filing: **19.01.2024**

(51) International Patent Classification (IPC):
*A61B 5/024* (2006.01)     *A61B 5/18* (2006.01)

(52) Cooperative Patent Classification (CPC):
**A61B 5/18; A61B 5/02405; A61B 5/02416**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Harman Becker Automotive Systems
GmbH
76307 Karlsbad (DE)**

(72) Inventors:
• **SHISHALOV, Ivan
76037 Karlsbad (DE)**
• **FILIMONOV, Andrei
76037 Karlsbad (DE)**
• **SHILOV, Andrey
76037 Karlsbad (DE)**
• **YOLYAN, Lilit
76037 Karlsbad (DE)**

(74) Representative: **Westphal, Mussgnug & Partner,
Patentanwälte mbB
Werinherstraße 79
81541 München (DE)**

(54) **CAMERA SYSTEM, METHOD FOR CONTROLLING A CAMERA SYSTEM, AND IMAGE EVALUATION UNIT COMPRISING A CAMERA SYSTEM**

(57)     A camera system comprises a camera (34) and a control unit (38), the camera (34) is configured to capture a sequence of successive images of a person of interest within a field of view of the camera (34), the camera system (30) is configured to determine an intensity of light upon the person of interest, and the control unit (38) configured to control camera settings of the camera (34) based on the intensity of light determined by the camera system (30), wherein the control unit (38) is configured to change the camera settings of the camera (34) only at defined points in time (tx1, tx2, ...txn), and, when it is detected that the intensity of light upon the object of interest changes between two defined points in time (tx1, tx2, ..., txn), current camera settings are maintained until the next defined point in time (tx1, tx2, ..., txn).

FIG 3

**Description**

TECHNICAL FIELD

The disclosure relate to a camera system, a related method for controlling a camera system, and an image evaluation unit comprising a camera system.

BACKGROUND

Physiological parameters are often determined in order to determine a mental state of a person, i.e. whether a person is drowsy, sleepy, or inattentive. Such information is often used, for example, in vehicles which may output a warning if it is determined that a person is not considered fit to drive. In this way, the risk of (fatal) accidents may be significantly decreased. Physiological parameters such as, e.g., a heart rate, pulse, blood pressure, breathing rate and breathing patterns, may be determined in many different ways. Some methods for determining physiological parameters require one or more sensors to be attached to the person that is to be monitored. This may be inconvenient, e.g., for a driver of a vehicle. A highly unobtrusive way for determining physiological parameters includes capturing images of the monitored person and determining physiological parameters by detecting slight changes in pixel intensity in subsequent images. Especially in moving vehicles, however, (rapidly) changing lighting conditions may generate unwanted noise which may negatively affect the accuracy of the physiological parameter determination.

There is a need for a camera system, a method for controlling a camera system, and an image evaluation unit comprising a camera system with which the influence of changing lighting conditions may be significantly reduced.

SUMMARY

A camera system includes a camera and a control unit, the camera is configured to capture a sequence of successive images of a person of interest within a field of view of the camera, the camera system is configured to determine an intensity of light upon the person of interest, and the control unit configured to control camera settings of the camera based on the intensity of light determined by the camera system, wherein the control unit is configured to change the camera settings of the camera only at defined points in time, and, when it is detected that the intensity of light upon the object of interest changes between two defined points in time, current camera settings are maintained until the next defined point in time.

An image evaluation unit includes the camera system and a physiological parameter determination unit, wherein the physiological parameter determination unit is configured to determine pixel intensity changes at least in defined regions of interest in the images captured by the camera system, and determine one or more physiological parameters of the person of interest based on the pixel intensity changes.

A method includes capturing, by means of a camera, a sequence of successive images of a person of interest within a field of view of the camera, determining an intensity of light upon the person of interest, and controlling, by means of a control unit, camera settings of the camera based on the determined intensity of light, wherein the camera settings of the camera are only changed at defined points in time, and, when it is detected that the intensity of light upon the object of interest changes between two defined points in time, current camera settings are maintained until the next defined point in time.

Other systems, features and advantages of the disclosure will be or will become apparent to one with skill in the art upon examination of the following detailed description and figures. It is intended that all such additional systems, methods, features and advantages included within this description be within the scope of the invention and be protected by the following claims.

BRIEF DESCRIPTION OF THE DRAWINGS

The arrangements and methods may be better understood with reference to the following description and drawings. The components in the figures are not necessarily to scale, emphasis instead being placed upon illustrating the principles of the invention. Moreover, in the figures, the same reference numerals designate the same components throughout the different views.

Figure 1 schematically illustrates a vehicle comprising an image evaluation unit according to embodiments of the disclosure.

Figure 2 schematically illustrates a method for determining a mental state of a person.

Figure 3 schematically illustrates a camera system according to embodiments of the disclosure.

Figure 4 schematically illustrates a vehicle travelling along a road.

Figure 5 schematically illustrates, in a flow diagram, a method according to embodiments of the disclosure

DETAILED DESCRIPTION

The camera system and related method for controlling a camera system are able to significantly reduce the influence of (rapidly) changing lighting conditions when determining physiological parameters of a person of interest on images captured by the camera system. When the images captured by the camera system are analyzed, e.g., by means of an image evaluation unit, in order to determine the physiological parameters of the person of interest (e.g., a driver of a vehicle), the results of the determination are very accurate.

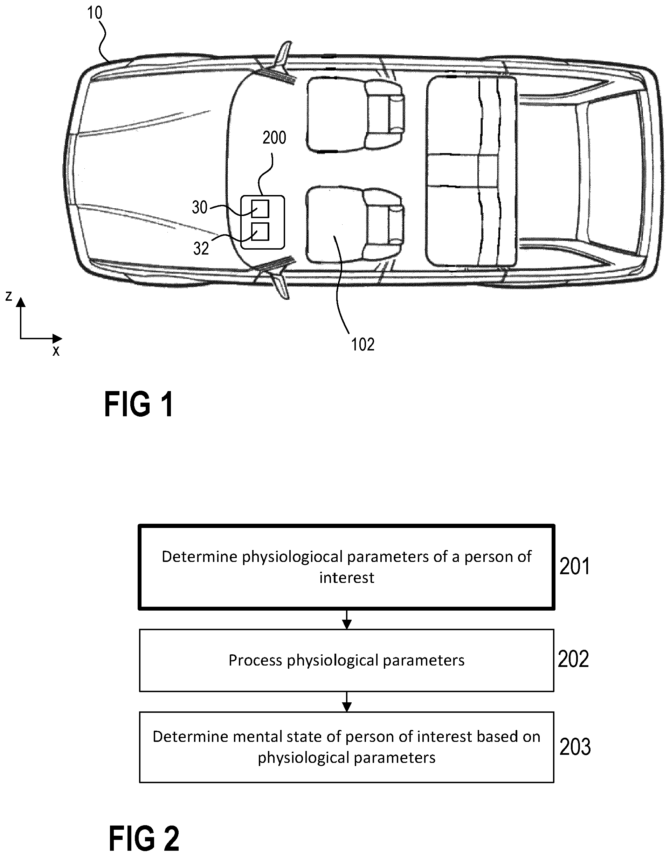

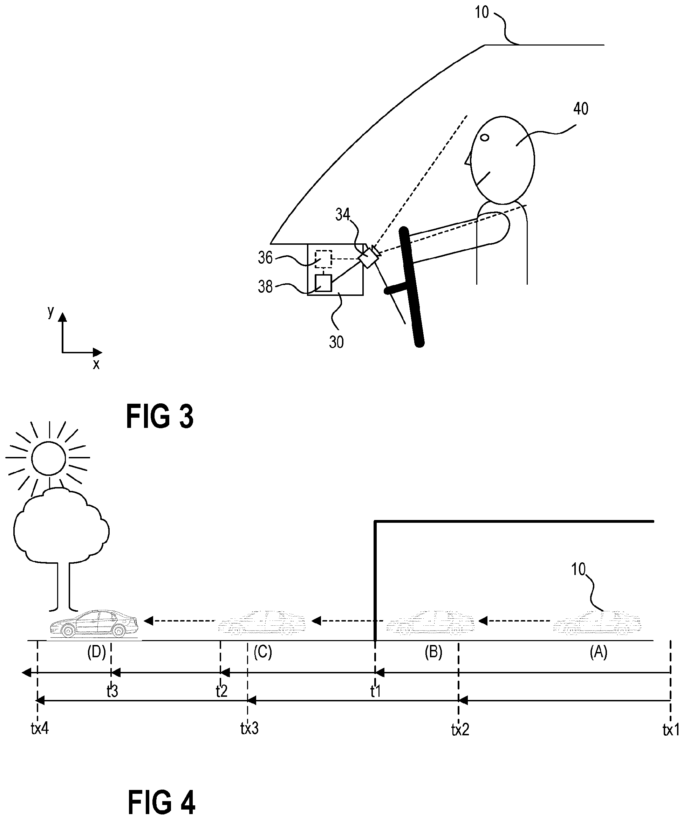

Figure 1 schematically illustrates a vehicle 10 comprising an image evaluation unit 200 according to embodiments of the disclosure. The image evaluation unit 200 comprises a camera system 30 and a physiological parameter determination unit 32. The camera system 30 comprises a camera 34 (see, e.g., Figure 3) and is configured to capture a sequence of successive images of a person of interest within a field of view of the camera 34. When arranged in a vehicle 10, the camera 34 of the camera system 30 may be directed towards a driver's seat 102 of the vehicle 10, for example. The camera 34 has a defined field of view, and by directing the camera 34 (i.e. the field of view of the camera 34) towards the driver's seat 102, the face of a driver 40 seated on the driver's seat 102 is within the field of view of the camera 34, as is schematically illustrated in Figure 3. The image evaluation unit 200 further comprises a physiological parameter determination unit 32 configured to determine pixel intensity changes at least in defined regions of interest in the images captured by the camera system 30, and determine one or more physiological parameters of the person of interest based on the pixel intensity changes

Physiological parameters are often determined in order to determine a mental state of a person, i.e. whether a person is drowsy, sleepy, or inattentive. Such information is often used, for example, in vehicles which may output a warning if it is determined that a person (i.e. the driver 40 of the vehicle 10) is not considered fit to drive. In this way, the risk of (fatal) accidents may be significantly decreased. Physiological parameters may include, e.g., a heart rate, pulse, blood pressure, breathing rate and breathing patterns. A highly unobtrusive way for determining physiological parameters includes capturing a plurality of images of the monitored person (person of interest) and determining one or more physiological parameters by detecting slight changes in pixel intensity in subsequent images.

Referring to Figure 2, a method for determining a mental state of a person (e.g., whether a driver 40 of a vehicle 10 is drowsy, sleepy, and/or inattentive) is schematically illustrated by means of a flow diagram. The method comprises determining physiological parameters (e.g., a heart rate and/or a heart rate variability) of a person of interest (step 201). The one or more physiological parameters are then processed (step 202), and a mental state of the person of interest is determined based on the one or more physiological parameters (step 203). The image evaluation unit 200 may be used to perform the first step (step 201) of such method. That is, the image evaluation unit 200 may be used to determine one or more physiological parameters of a person of interest. Optionally, the image evaluation unit 200 may also perform the subsequent steps of the method (steps 202 and 203). Such subsequent steps, however, may instead be performed by other units that are separate and distinct from the image evaluation unit 200.

Methods for determining physiological parameters of a person of interest by evaluating images captured by means of a camera 34 are generally known. Such methods, however, may be imprecise, as they include determining one more physiological parameters by detecting slight changes in pixel intensity in subsequent images. Especially in moving vehicles, (rapidly) changing lighting conditions may generate unwanted noise which may negatively affect the accuracy of the physiological parameter determination. This is described in more detail with respect to Figure 4, which schematically illustrates a vehicle 10 moving along a road. For example, as is indicated on the right side of Figure 4, a vehicle 10 may drive through a tunnel. While driving through the tunnel (vehicle positions (A) and (B)), lighting conditions may be poor and camera settings have to be applied that match the poor lighting conditions. For example, a comparably long exposure time and a low f-number may be set by the control unit 38. As long as the vehicle 10 is travelling through the tunnel, the lighting conditions may be essentially stable. However, due to irregular illumination within a tunnel, lighting conditions may even change to a certain degree within the tunnel. For example, lighting conditions may be somewhat better when the vehicle 10 is passing a lamp, and may be somewhat poorer when the vehicle 10 is in between two subsequent lamps. When the vehicle 10 leaves the tunnel, lighting conditions may significantly change, especially when there is bright sunshine outside the tunnel. However, the vehicle 10, when outside of the tunnel, may also pass objects such as trees or buildings, for example, such that lighting conditions may rapidly change between bright sunshine (vehicle position (C)) and shade (vehicle position (D)).

Each time the lighting conditions change, camera settings will be (automatically) adjusted in order to avoid underexposed or overexposed images. Camera settings that may be adjusted in order to avoid underexposure or overexposure may include, e.g., automatic iris adjustment, where the aperture controls the flow of light from the camera's optical subsystem to the photosensitive elements. Reducing the aperture reduces the light flow to the light-sensitive elements of the camera 34, thus reducing the brightness of the pixels in the case of digital cameras. Additionally or alternatively, camera settings that may be adjusted in order to avoid underexposure or overexposure may include, e.g., automatic exposure control. Exposure, or the time the light-sensitive elements are exposed to incoming light, determines the total amount of energy that reaches the light-sensitive elements and, consequently, the brightness of the pixels. Exposure time and f-number are generally related to automatic exposure control. Additionally or alternatively, camera settings that may be adjusted in order to avoid underexposure or overexposure may include various other techniques for analog or digital amplification of a signal received from the light-sensitive elements in order to obtain adequate pixel brightness values in terms of perception of human beings or processing algorithms. In general, any of the above mentioned approaches "multiplies" the pixel brightness by a coefficient, and the result is either amplification or attenuation of the resulting signal. Generally speaking, the use of any of such adjustment techniques results in the elimination of the effects of underexposure in cases where the pixels of the image are too dark and, therefore, unsuitable for further analysis due to low brightness or indistinguishability in relation to each other, or overexposure in cases where the pixels are too bright and equally unsuitable for further analysis due to strong brightness and indistinguishability in relation to each other, which provides an acceptable dynamic range of the captured objects.

Changes in pixel intensity caused by a change of lighting conditions and a resulting change of camera settings are generally significantly larger than changes in pixel intensity caused by a change of skin color due to blood pulsating in the person of interest. Automatically adjusting the camera settings leads to a sharp change of properties (e.g., increase or decrease of brightness) of all or at least a subset of pixels in the captured images. The amplitudes of the changes are comparably large, in many cases much larger than one unit of pixel brightness. Heartrate estimation systems generally calculate the change in skin reflectance caused by the pulsation of blood in the blood vessels. The pulsation results in changes in the "color" and pixel intensity of portions of the image representing the skin. Such systems can be based on both digital signal processing algorithms and systems using machine learning and artificial neural networks. With a standard level of camera technology, such changes in pixel intensity caused by a change of skin color due to blood pulsating in the person of interest often hardly match the dynamic range of the camera 34. In other words, pixel intensity changes related to physiological parameter determination generally have an amplitude that is comparable or even less than a single unit of pixel brightness change. For example, with a standard camera luminance component encoding of 8 bits, corresponding to a luminance range of 0 - 255, such changes are often less than $1/255^{th}$ of the luminance range defined by the camera, or 1 unit. Averaging and using information from all related pixels of the image (or at least of a subset of pixels) generally provides the possibility of building systems with sufficient characteristics for useful applications. An algorithm evaluating the pixel intensity in order to determine physiological parameters of the person of interest is no longer able to deliver reliable results when lighting conditions change rapidly and camera settings are rapidly adjusted accordingly.

Changes of pixel brightness that are caused by an adjustment of camera settings and not by the monitored physiological parameter itself are generally considered as noise. As described above, this noise may be significantly greater in amplitude than the changes caused by the monitored physiological parameter. The noise caused by an adjustment of camera settings is generally temporal and, therefore, cannot be (easily) eliminated by suitable processing techniques (e.g., by averaging pixel brightness over a single image).

A camera system 30 according to embodiments of the disclosure (see, e.g., Figure 3) comprises a camera 34 and a control unit 38. The camera 34 is configured to capture a sequence of successive images of a person of interest within a field of view of the camera 34. The camera system 30 is configured to determine an intensity of light upon the person of interest, and the control unit 38 is configured to control camera settings of the camera 34 based on the intensity of light determined by the camera system 30. In order to reduce the effects of the noise caused by automatic adjustment of camera settings, the control unit 38 is configured to change the camera settings of the camera 34 only at defined points in time tx1, tx2, ... txn. When it is detected that the intensity of light upon the person of interest changes between two defined points in time tx1, tx2, ..., txn, current camera settings are maintained until the next defined point in time tx1, tx2, ..., txn. The intensity of light upon the person of interest may be determined in any suitable way. Most cameras today may determine the intensity of light upon a person (or object) of interest, e.g., by means of suitable sensors and evaluation circuitry. For example, an intensity of light upon a person of interest may be determined by suitably evaluating camera parameters such as, e.g., pixel brightness, exposure time, iris, amplification, etc. According to another example, a camera 34 included in the camera system 30 may have an integrated exposure meter, light meter or luminosity meter configured to determine an intensity of light upon the person of interest. It is, however, also possible that the camera system 30 comprises an exposure meter, light meter or luminosity meter 36 that is separate from the camera 34.

Referring to Figure 4, for example, automatic adjustment of camera settings would be performed in conventional systems at time instant 11, when the vehicle 10 leaves the tunnel and lighting conditions change. Time instant t1 in the present example, however, does not correspond to any of the defined points in time tx1, tx2, tx3, tx4 (and so on). In order to avoid too many changes of camera settings within a short timeframe (e.g., a change of camera settings every 1 to 4 seconds), the camera settings remain the same irrespective of whether lighting conditions change until the next defined point in time tx1, tx2, ...txn. That is, in the example illustrated in Figure 4, camera settings would only change at time instant tx3. Further changes at time instants t2 and t3, due to changing lighting conditions would also be suppressed. The next change of camera settings would happen at time instant tx4. Between time instant tx3 and time instant tx4, therefore, there may be underexposed images (due to the shading caused by the tree in the present example). In other situations, there may also be overexposed images, until the camera settings are changed again at the respective next defined point in time tx1, tx2, ... txn.

The defined points in time tx1, tx2, ...txn at which a change of camera settings may be performed are known to the physiological parameter determination unit 32 of an image evaluation unit 200. The evaluation algorithm that is used for determining the physiological parameters based on the captured images may pause momentarily at the defined points in time tx1, tx2, ... txn or a sensitivity of the evaluation algorithm may be reduced at the defined points in time tx1, tx2, ... txn, for example. In this way, any influences caused by a change of the camera settings may be significantly reduced. Between two subsequent defined points in time tx1, tx2, ... txn, there is an interval of image parameter stability (image parameters/camera settings are stable), which significantly increases the accuracy of the physiological parameter determination based on the captured images. The interval between two directly successive defined points in time tx1, tx2, ..., txn may be at least 10 seconds, at least 15 seconds, or at least 30 seconds, for example. Generally, increasing a time between two directly successive defined points in time tx1, tx2, ... txn results in a simplification of an internal structure of the noise. This allows to filter unwanted noise more efficiently.

As has been described above, the evaluation algorithm may rely on the fact that camera settings are fixed during the intervals between two successive defined points in time tx1, tx2, ..., txn. It is, however, generally also possible that the evaluation algorithm compensates for any changes of camera settings made at a defined point in time tx1, tx2, ..., txn. For example, the control unit 38 may provide information about any changes it made to the camera settings to the physiological parameter determination unit 32. The physiological parameter determination unit 32 may then take into account this information when determining the physiological parameters. That is, in addition to the images captured by means of the camera 34, the camera system 30 may provide additional information concerning the camera settings to the physiological parameter determination unit 32 for further consideration when determining the physiological parameters.

It is generally possible, that the intervals between two successive defined points in time tx1, tx2, ... txn are chosen to be long. For example, an interval between two directly successive defined points in time tx1, tx2, ..., txn may be several minutes, or even hours. For example, camera settings may not be changed at all during an entire driving session of a vehicle. Camera settings may be set once at the beginning of a driving session and may be maintained for the rest of the journey. In this case, the camera settings may be considered constant. Automatic camera setting adjustment may be essentially disabled. In this way, noise that is caused by camera setting adjustment is entirely eliminated. When setting the intervals between two successive defined points in time tx1, tx2, ...txn too long, however, this may result in significant underexposure and/or overexposure for many images when lighting conditions change often. It may then no longer be possible to accurately detect a head position of the person of interest, identify the person of interest by means of suitable face recognition algorithms, etc. That is, certain disadvantages would have to be accepted if the time interval between two subsequent defined points in time tx1, tx2, ..., txn is chosen to be long.

The control unit 38, in some cases may not be able to provide information about a change of the camera settings to the physiological parameter determination unit 32. In such cases, the image evaluation unit 200 may be configured to estimate the changes that were made to the camera settings by means of suitable algorithms. The defined points in time tx1, tx2, ...txn are still known. The image evaluation unit 200, therefore, may estimate the changes that were made to the camera settings by suitably evaluating an image captured directly before, and an image captured directly after a defined point in time tx1, tx2, ..., txn. According to one example, the image evaluation unit 200 may comprise or may be coupled to a near infrared camera that is configured to generate near infrared flashes. Such flashes are not visible for the person of interest. The image evaluation unit 200, however, knows the characteristics of the artificial light generated to produce the flashes. Reference objects may be arranged close to the person of interest. For example, reference objects of a defined color (e.g., white) may be attached to or integrated in a headrest of a vehicle 10. The head of the driver 40 of the vehicle 10 would then be directly adjacent to such reference objects (e.g., simple white circles on a headrest, or entirely white headrest), and both the head of the person of interest as well as the reference objects (or at least one reference object) will be captured on the images. The characteristics (e.g., specific color) of the reference objects are known to the image evaluation unit 200. Based on the known characteristics of the near infrared flashes, the known characteristics of the reference objects, and the captured images, the image evaluation unit 200 may then determine a change of camera settings by means of suitable algorithms. In other words, the image evaluation unit 200 estimates the camera settings from what the reference objects look like (e.g., bright or dark) in the captured images. Instead of on a headrest, reference objects could be arranged on any other suitable elements within a vehicle (e.g., on a part of a steering wheel that is visible in the images, or on a part of a backrest of a seat that is visible in the images, etc.).

As described above, it is possible that the algorithm that is used in order to determine the physiological parameters compensates for any changes of camera settings made at a defined point in time tx1, tx2, ..., txn. An example of how such a compensation may be implemented will be provided in the following. The influence of a change of camera settings is generally sufficiently predictable, at least in a first approximation. For example, increasing the exposure by a factor of two, leads to a twofold increase in the total energy received by the photosensitive elements, which leads to a twofold increase in their average brightness. The same statement is true for gain control and iris opening.

If the exposure value for a frame f is E(f), aperture is D(f), and gain is G(f), then their change between frames f1 and f2 will be respectively:

$$C_e(f_1, f_2) = \frac{E(f_2)}{E(f_1)}, C_d(f_1, f_2) = \frac{D(f_2)}{D(f_1)}, C_g(f_1, f_2) = \frac{G(f_2)}{G(f_1)} \qquad (1)$$

The total coefficient of variation in pixel brightness due to tweaking (optimization) would then be:

$$C(f_1, f_2) = C_e(f_1, f_2)C_d(f_1, f_2)C_g(f_1, f_2) \qquad (2)$$

If the arrangement allows obtaining exposure, aperture, gain, and other parameters affecting the discussed changes in pixel brightness, such parameters can be used for calculating the brightness correction factor. For an arbitrarily taken pixel of an image $P(x, y, f)$ with coordinates (x,y) at frame f, it is possible to obtain normalized brightness, normalized, for example, to the first frame in the video stream:

$$P'(x, y, f) = \frac{P(x,y,f)}{C(1,f)} \qquad (3)$$

As can be seen from formulas (1), (2), and (3) above, this normalization results in compensation for the variation in pixel brightness due to parameter tweaking and makes the image stable with respect to such variations.

Possible extensions or generalizations of the compensation are:

- Normalization not to the first frame, but to another frame, chosen by means of an internal logic of the physiological parameter determination algorithm, for example, bound to a moment of algorithm calibration;

- Periodic selection of a frame for normalization (e.g., when a reference frame for the normalization is selected every 10 seconds);

- As an extreme case of the previous extensions, inter-frame compensation, when the difference in between two consequent frames is compensated:

$$P'(x, y, f) = \frac{P(x,y,f)}{C(f-1,f)} \qquad (4)$$

- Consideration of nonlinear effects of the influence of parameter changes on the change of pixel brightness. In the general case and depending on the realization, the dependence of the influence of parameter values on brightness will have the following form:

$$P(x, y, f) = G(E(D(e(x, y, f), D), E), G) \qquad (5)$$

Where P(x,y,f) is the brightness of pixel (x,y) in frame f, e(x,y,f) is the amount of energy entering the camera and attributable to pixel (x,y) during the capture of frame f, D(e,D) is the energy reduction factor e at aperture D, E(e, E) is the energy reduction factor e at exposure value E, G is a similar factor for gain. As e(x,y,f) can be taken as normalized pixel brightness, and as it does not depend on camera parameters:

$$e(x, y, f) = D^{-1}(E^{-1}(G^{-1}(P(x, y, f), G), E), D) \qquad (6)$$

The camera system 30 and image evaluation unit 200 have been described as being arranged in a vehicle 10 above. In vehicles 10, physiological parameters of a driver 40 or of any other passengers may be determined for different applications, e.g., in order to determine a mental state of the driver 40 (i.e. whether the driver 40 is fit to drive). In vehicles 10, lighting conditions may change rapidly. It is, however, generally possible that the camera system 30 and image evaluation unit 200 are used in any other environment than a vehicle.

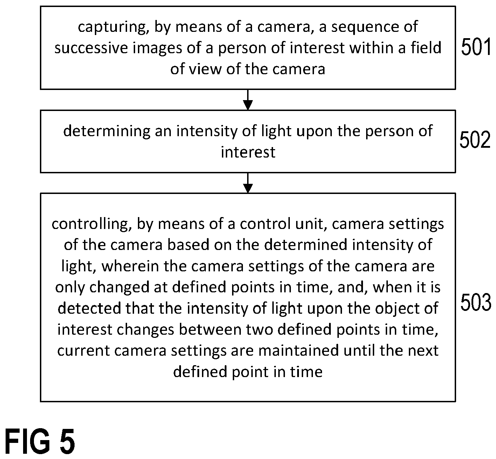

A method according to embodiments of the disclosure, as is schematically illustrated in Figure 5, comprises capturing, by means of a camera 34, a sequence of successive images of a person of interest within a field of view of the camera 34 (step 501), determining an intensity of light upon the person of interest (step 502), and controlling, by means of a control unit 38, camera settings of the camera 34 based on the determined intensity of light (step 503), wherein the camera settings of the camera 34 are only changed at defined points in time tx1, tx2, ...txn, and, when it is detected that the intensity of light upon the object of interest changes between two defined points in time tx1, tx2, ..., txn, current camera settings are maintained until the next defined point in time tx1, tx2, ..., txn.

The method may further include determining pixel intensity changes at least in defined regions of interest in the captured images, and determining one or more physiological parameters of the person of interest based on the pixel intensity changes. The one or more physiological parameters may subsequently be processed, and a mental state of the person of interest may be determined based on the physiological parameters. The one or more physiological parameters may include at least one of a heart rate and a heart rate variability, for example.

It may be understood, that the illustrated systems and methods are merely examples. While various embodiments of the invention have been described, it will be apparent to those of ordinary skill in the art that many more embodiments and implementations are possible within the scope of the invention. In particular, the skilled person will recognize the interchangeability of various features from different embodiments. Although these techniques and systems have been disclosed in the context of certain embodiments and examples, it will be understood that these techniques and systems may be extended beyond the specifically disclosed embodiments to other embodiments and/or uses and obvious modifications thereof. Accordingly, the invention is not to be restricted except in light of the attached claims and their equivalents.

The description of embodiments has been presented for purposes of illustration and description. Suitable modifications and variations to the embodiments may be performed in light of the above description or may be acquired from practicing the methods. The described arrangements are exemplary in nature, and may include additional elements and/or omit elements. As used in this application, an element recited in the singular and proceeded with the word "a" or "an" should be understood as not excluding plural of said elements, unless such exclusion is stated. Furthermore, references to "one embodiment" or "one example" of the present disclosure are not intended to be interpreted as excluding the existence of additional embodiments that also incorporate the recited features. The terms "first," "second," and "third," etc. are used merely as labels, and are not intended to impose numerical requirements or a particular positional order on their objects. The subject matter of the present disclosure includes all novel and non-obvious combinations and sub-combinations of the various systems and configurations, and other features, functions, and/or properties disclosed. The following claims particularly point out subject matter from the above disclosure that is regarded as novel and non-obvious.

## Claims

1. A camera system (30) comprises a camera (34) and a control unit (38),

   the camera (34) is configured to capture a sequence of successive images of a person of interest within a field of view of the camera (34);
   the camera system (30) is configured to determine an intensity of light upon the person of interest; and
   the control unit (38) is configured to control camera settings of the camera (34) based on the intensity of light determined by the camera system (30), wherein
   the control unit (38) is configured to change the camera settings of the camera (34) only at defined points in time (tx1, tx2, ...txn), and, when it is detected that the intensity of light upon the object of interest changes between two defined points in time (tx1, tx2, ..., txn), current camera settings are maintained until the next defined point in time (tx1, tx2, ..., txn).

2. The camera system (30) of claim 1, wherein an interval between two directly successive defined points in time (tx1, tx2, ..., txn) is at least 10 seconds, at least 15 seconds, or at least 30 seconds.

3. The camera system (30) of claim 1 or 2, wherein the camera settings controlled by the control unit (38) include at least one of an exposure time and a f-number.

4. An image evaluation unit (200) comprises,

   a camera system (30) of any of claims 1 to 3; and
   a physiological parameter determination unit (32), wherein
   the physiological parameter determination unit (32) is configured to determine pixel intensity changes at least in defined regions of interest in the images captured by the camera system (30), and determine one or more physiological parameters of the person of interest based on the pixel intensity changes.

5. The image evaluation unit (200) of claim 4, wherein the one or more physiological parameters determined by the physiological parameter determination unit (32) include at least one of a heart rate and a heart rate variability.

6. The image evaluation unit (200) of claim 4 or 5, wherein the physiological parameter determination unit (32) is configured to determine the one or more physiological parameters by means of an evaluation algorithm, wherein the algorithm is paused momentarily at the defined points in time (tx1, tx2, ... txn), or a sensitivity of the algorithm is reduced at the defined points in time (tx1, tx2, ... txn).

7. The image evaluation unit (200) of claim 6, wherein

the physiological parameter determination unit (32) receives the images captured by the camera (34) of the camera system (30);
the physiological parameter determination unit (32) further receives information from the control unit (38) of the camera system (30) concerning changes the control unit (38) made to the camera settings at the defined points in time (tx1, tx2, ..., txn); and
the evaluation algorithm compensates for changes of camera settings made at the defined points in time (tx1, tx2, ..., txn).

8. The image evaluation unit (200) of claim 6, wherein

the physiological parameter determination unit (32) receives the images captured by the camera (34) of the camera system (30);
the image evaluation unit (200) is configured to estimate changes the control unit (38) made to the camera settings at the defined points in time (tx1, tx2, ..., txn); and
the evaluation algorithm compensates for changes of camera settings made at the defined points in time (tx1, tx2, ..., txn).

9. The image evaluation unit (200) of claim 8, wherein the image evaluation unit (200) is configured to estimate the changes that were made to the camera settings by evaluating an image captured directly before, and an image captured directly after a defined point in time (tx1, tx2, ..., txn).

10. The image evaluation unit (200) of any of claims 4 to 9, wherein the image evaluation unit (200) is arranged in a vehicle (10), and the person of interest is a driver (40) of the vehicle (10).

11. A method comprises

capturing, by means of a camera (34), a sequence of successive images of a person of interest within a field of view of the camera (34);
determining an intensity of light upon the person of interest; and
controlling, by means of a control unit (38), camera settings of the camera (34) based on the determined intensity of light, wherein
the camera settings of the camera (34) are only changed at defined points in time (tx1, tx2, ...txn), and, when it is detected that the intensity of light upon the object of interest changes between two defined points in time (tx1, tx2, ..., txn), current camera settings are maintained until the next defined point in time (tx1, tx2, ..., txn).

12. The method of claim 11, further comprising

determining pixel intensity changes at least in defined regions of interest in the captured images; and
determining one or more physiological parameters of the person of interest based on the pixel intensity changes.

13. The method of claim 12, further comprising

processing the one or more physiological parameters; and
determining a mental state of the person of interest based on the physiological parameters.

14. The method of any of claims 11 to 13, wherein the one or more physiological parameters include at least one of a heart rate and a heart rate variability.

**FIG 1**

| Determine physiologiocal parameters of a person of interest | 201 |
| Process physiological parameters | 202 |
| Determine mental state of person of interest based on physiological parameters | 203 |

**FIG 2**

**FIG 3**

**FIG 4**

capturing, by means of a camera, a sequence of successive images of a person of interest within a field of view of the camera | 501

determining an intensity of light upon the person of interest | 502

controlling, by means of a control unit, camera settings of the camera based on the determined intensity of light, wherein the camera settings of the camera are only changed at defined points in time, and, when it is detected that the intensity of light upon the object of interest changes between two defined points in time, current camera settings are maintained until the next defined point in time | 503

# FIG 5

Europäisches
Patentamt

European
Patent Office

Office européen
des brevets

# EUROPEAN SEARCH REPORT

Application Number

EP 24 15 2828

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/327800 A1 (CHEN TUNG-CHIEN [TW] ET AL) 19 November 2015 (2015-11-19) * paragraphs [0024], [0025], [0027]; claims 1, 4, 6, 9, 13, 15, 18, 23, 27; figures 2, 4, 5 * | 1-14 | INV. A61B5/024 A61B5/18 |
| A | HUANG PO-WEI ET AL: "A Heart Rate Monitoring Framework for Real-World Drivers Using Remote Photoplethysmography", IEEE JOURNAL OF BIOMEDICAL AND HEALTH INFORMATICS, IEEE, PISCATAWAY, NJ, USA, vol. 25, no. 5, 24 September 2020 (2020-09-24), pages 1397-1408, XP011853827, ISSN: 2168-2194, DOI: 10.1109/JBHI.2020.3026481 [retrieved on 2021-05-10] * paragraph [0001] - paragraph [0002] * | 10,13 | |

**TECHNICAL FIELDS SEARCHED (IPC)**

A61B

The present search report has been drawn up for all claims

| Place of search | Date of completion of the search | Examiner |
|---|---|---|
| The Hague | 3 May 2024 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 2828

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-2024

| Patent document cited in search report | Publication date | Patent family member(s) | Publication date |
|---|---|---|---|
| US 2015327800 A1 | 19-11-2015 | CN 105078407 A | 25-11-2015 |
| | | EP 2945368 A1 | 18-11-2015 |
| | | US 2015327800 A1 | 19-11-2015 |

EPO FORM P0459

For more details about this annex : see Official Journal of the European Patent Office, No. 12/82